(19) Europäisches Patentamt
European Patent Office
Office européen des brevets

(11) Veröffentlichungsnummer: **0 054 168**
**B1**

(12) # EUROPÄISCHE PATENTSCHRIFT

(45) Veröffentlichungstag der Patentschrift: **11.07.84**

(51) Int. Cl.³: **C 07 C 93/06**, A 61 K 31/135

(21) Anmeldenummer: **81109254.3**

(22) Anmeldetag: **29.10.81**

(54) **1,1,2-Triphenyl-but-1-en-Derivate sowie Verfahren zu ihrer Herstellung und ihre Verwendung als Arzneimittel.**

(30) Priorität: **11.12.80 DE 3046719**

(43) Veröffentlichungstag der Anmeldung: **23.06.82 Patentblatt 82/25**

(45) Bekanntmachung des Hinweises auf die Patenterteilung: **11.07.84 Patentblatt 84/28**

(84) Benannte Vertragsstaaten: **AT BE CH DE FR GB IT LI LU NL SE**

(56) Entgegenhaltungen: **DE - A - 1 493 282**

(73) Patentinhaber: **Klinge Pharma GmbH, Berg-am-Laim-Strasse 129, D-8000 München 80 (DE)**

(72) Erfinder: **Schickaneder, Helmut, Dr., Anzinger Strasse 22, D-8011 Poing (DE)**
Erfinder: **Löser, Roland, Dr., Fichtenweg 12, D-8133 Feldafing (DE)**
Erfinder: **Grill, Helmut, Dr., Zugspitzstrasse 148, D-8011 Vaterstetten (DE)**

(74) Vertreter: **Wuesthoff, Franz, Dr.-Ing. et al, Patentanwälte Wuesthoff -v. Pechmann-Behrens-Goetz Schweigerstrasse 2, D-8000 München 90 (DE)**

EP 0 054 168 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft neue 1,1,2-Triphenyl-but-1-en-Derivate, welche wertvolle therapeutische Eigenschaften besitzen.

Verbindungen mit diesem Grundgerüst und einem Dialkylaminoalkoxyrest in Paraposition an einem der Phenylreste am C-Atom 1 der But-1-en-kette, sind bereits in der Britischen Patentschrift 1 013 907 beschrieben. Eine davon, das (Z)-1- [4'-(2-Dimethylaminoethoxy)- phenyl]-1,2-diphenyl-but-1-en (Tamoxifen, INN rec.) stellt einen spezifischen Estrogen-Antagonist dar. Aufgrund seiner ausgeprägten antiestrogenen Aktivität hat sich dieser Wirkstoff bereits in der Therapie des hormonabhängigen Mammatumors bewährt.

In der DE-OS 2 807 599 ist festgestellt worden, dass ein Metabolit des Tamoxifens, das (Z)-1-[4'-(2- Dimethylaminoethoxy)phenyl]-1-(4'-hydroxyphenyl)-2-phenyl-but-1-en («4-Hydroxy-Tamoxifen») eine dem Tamoxifen vergleichbare antiestrogene Wirkung besitzt. Wie aus der Europ. Anmeldung 0 002 097 hervorgeht, trifft dies auch auf eine Reihe von 1-[4'-(2-Alkylaminoalkoxy)phenyl]-1-(4'-hydroxyphenyl)-2-phenylbut-1-enen («4-Hydroxy-Tamoxifen-Derivate») zu.

Überraschenderweise wurde nun festgestellt, dass durch Verschiebung der Hydroxygruppe von Position 4 nach Position 3 Verbindungen erhalten werden, deren E-Formen hinsichtlich Bindungsaffinität zum Estrogenrezeptor dem Tamoxifen deutlich überlegen sind. Aufgrund dieser hohen spezifischen Affinität zum Estrogenrezeptor zeigten die beanspruchten Verbindungen erwartungsgemäss eine sowohl ausgeprägte antiuterotrope Aktivität als auch mammatumorhemmende Wirkung, die, wie am Beispiel der Verbindung 1 gezeigt wird, über der Aktivität des Tamoxifens lag. Gegenstand der Erfindung sind 1,1,2-Triphenyl-but-1-en-Derivate der allgemeinen Formel (1), deren Konfiguration der E-Form entspricht.

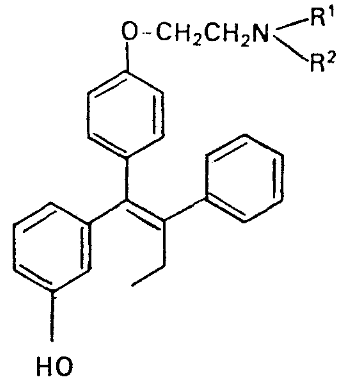

(1)

Folgende Verbindungen werden beansprucht:

Tabelle 1

| Verb. Nr. | $R^1$ | $R^2$ | Schmp. | Beispiel |
|---|---|---|---|---|
| 1 | $CH_3$ | $CH_3$ | 162 bis 163 °C | 1 h |
| 2 | $C_2H_5$ | $C_2H_5$ | 121 °C | 2 a |
| 3 | $CH_3$ | H | 125 bis 127 °C | 3 |
| 4 | $C_2H_5$ | H | 174 bis 175 °C | 4 |

In dieser Beschreibung beziehen sich die Bezeichnungen E- bzw. Z-Form (E= «entgegen», Z=«zusammen») auf die Stellung der 3-Hydroxyphenylgruppe (Priorität 1) am C-Atom 1 zur Stellung der unsubstituierten Phenylgruppe (Priorität 1) am C-Atom 2 der Doppelbindung zueinander [Nomenklaturregel: R.T. Morrison, R.N. Boyd, Lehrbuch der Organischen Chemie, Verlag Chemie, S. 167 (1974)].

Die E- und Z-Formen unterscheiden sich deutlich in ihren Protonenresonanzsignalen der Alkylaminogruppe und der $-O-CH_2$-Gruppe in der $-OCH_2CH_2NR^1R^2$-Seitenkette. Die Signale der E-Form sind bei den beanspruchten Verbindungen gegenüber der Z-Form hochfeldverschoben [D.J. Collins. J.J. Hobbs und C.W. Emmers, J. Med. Chem., 14, 952 (1971)].

Tabelle 2
Charakteristische Unterscheidungsmerkmale der E- und Z-Formen von 1-[4'-(2-Dialkylaminoethoxy)phenyl]-1-(3'-hydroxyphenyl)-2-phenyl-but-1-en-Verbindungen

| $R^1 = R^2$ | Isomer | Schmp. | $^1$H-NMR-Signale (δ, ppm) | |
|---|---|---|---|---|
| Methyl (Beispiel 1 h) | E-Form | 162 bis 163 °C | $N(CH_3)_2$<br>$OCH_2$ | 2.17<br>3.88 |
| Methyl (Beispiel 1 i) | Z-Form | 173 °C | $N(CH_3)_2$<br>$OCH_2$ | 2.23<br>4.05 |

| | | | | |
|---|---|---|---|---|
| Ethyl (Beispiel 2 a) | E-Form | 121 °C | $N(CH_2C\underline{H}_3)_2$<br>$OC\underline{H}_2$ | 0.97<br>3.90 |
| Ethyl (Beispiel 2 b) | Z-Form | 156 °C | $N(CH_2C\underline{H}_3)_2$<br>$OC\underline{H}_2$ | 1.01<br>4.03 |

Die Erfindung betrifft ferner ein Verfahren zu Herstellung von Verbindungen der allgemeinen Formel (1), das dadurch gekennzeichnet ist, dass man Carbinole der allgemeinen Formel (2)

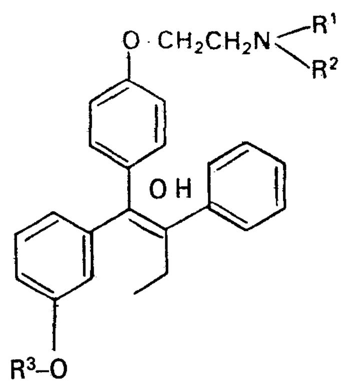

(2)

in der $R^1$ und $R^2$ die in Anspruch 2 angegebene Bedeutung besitzen und $R^3$ Wasserstoff oder eine leicht hydrolysierbare Schutzgruppe darstellt, in an sich bekannter Weise durch Einwirken von Mineralsäure, gegebenenfalls unter Abspaltung der Schutzgruppe, dehydratisiert, vom anfallenden Isomerenpaar die E-Form durch Kristallisation isoliert, und eine gegebenenfalls vorhandene Benzylgruppe hydrogenolytisch abspaltet. Als leicht hydrolysierbare Schutzgruppe wird der Tetrahydropyranylrest bevorzugt. Die Abspaltung der Schutzgruppe und die Dehydratisierung gelingt mit Mineralsäure in alkoholischem Milieu, bevorzugt in salzsaurer ethanolischer Lösung. Die Isolierung der E-Form mittels Kristallisation kann sowohl mit den Säure-Additions-Salzen als auch mit den freien Basen durchgeführt werden. Eine eventuelle Entbenzylierung gelingt selektiv hydrogenolytisch bei Raumtemperatur mit Palladium-Kohle.

Die erfindungsgemässen Verbindungen kann man beispielsweise wie folgt herstellen:

Die Ausgangsverbindung der Formel (3)

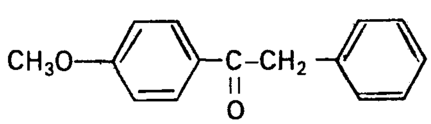

(3)

kann durch Friedel-Crafts-Reaktion von Methoxybenzol mit Phenylessigsäurechlorid erhalten werden. Durch Umsetzung von 1-(4'-Methoxyphenyl)-2-phenyl)-ethan-1-on (3) mit Ethylbromid in Dimethylformamid und in Gegenwart von Natriumhydrid entsteht das 1-(4'-Methoxyphenyl)-2-phenyl-n-butan-1-on (4)

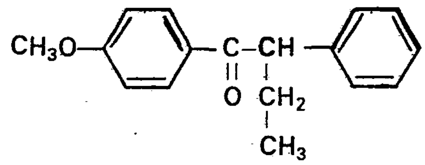

(4)

Eine Etherspaltung von (4) mit Pyridinhydrochlorid führt zum 1-(4'-Hydroxyphenyl)-2-phenyl-n-butan-1-on (5)

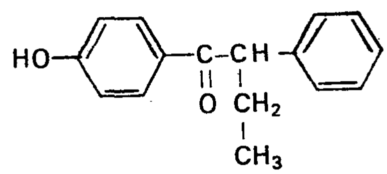

(5)

Durch Umsetzung von (5) mit Verbindungen der allgemeinen Formel $R^1R^2N-CH_2CH_2Cl$ (6) in der $R^1$ und $R^2$ gleich oder verschieden sein können, wobei im Falle $R^1$ gleich $R^2$ die Substituenten jeweils Methyl- oder Ethylgruppen bedeuten, während im Falle $R^1$ ungleich $R^2$ ein Substituent eine Benzylgruppe bedeutet und der andere Substituent eine Methyl- oder Ethylgruppe darstellt, werden Verbindungen der allgemeinen Formel (7) erhalten, wobei $R^1$ und $R^2$ die oben genannten Bedeutungen besitzen.

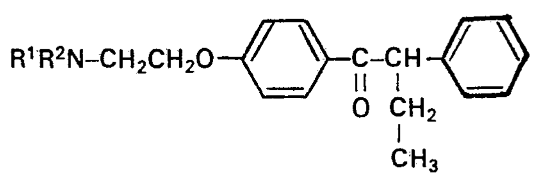

(7)

Verbindungen der allgemeinen Formel (7) werden mit 3'-(2-Tetrahydropyranyloxy)-phenyl-magnesiumbromid zu den diastereomeren Carbinolen der allgemeinen Formel (8) umgesetzt,

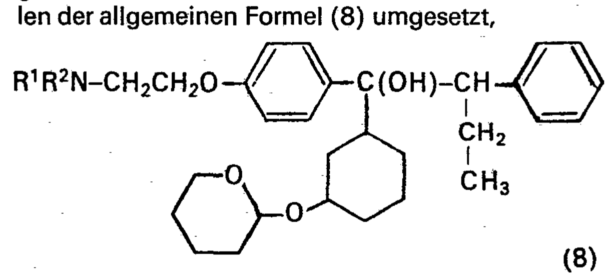

(8)

wobei $R^1$ und $R^2$ die in Formel (7) angegebene Bedeutung besitzen. Verbindungen der allgemeinen Formel (8) spalten in Gegenwart von Mineralsäure bereits bei Raumtemperatur den Tetrahydropyranylrest ab und dehydratisieren in der Hitze zu einem Isomerenpaar, aus dem sowohl in seiner Salz- als auch in seiner Basenform das E-Isomere durch Kristallisation isoliert werden kann, das gegebenenfalls bei Anwesenheit einer Benzylgruppe hydrogenolytisch entbenzyliert wird, so dass Verbindungen der allgemeinen Formel (1) nach Anspruch 1 erhalten werden.

In den durchgeführten Untersuchungen konnte bei den beanspruchten Verbindungen eine hohe antiestrogene Wirkung festgestellt werden, die therapeutisch nutzbar ist.

Die Bestimmung der Bindungsaffinität zum Estradiolrezeptor wurde mit dem Kaninchenuterus-Cytosol vorgenommen. Die beanspruchten Verbindungen zeigten gegenüber dem Tamoxifen eine ca. zehnfach höhere Bindungsaffinität.

Die Messung der antiuterotropen Wirkung erfolgt nicht, wie üblich, nach dreitägiger Wirkstoffbehandlung von präpubertären weiblichen Ratten (Dorfman-Test). Innerhalb dieser kurzen Behandlungszeit ist eine hormonelle Gegenregulation bei präpubertären Tieren nicht zu erwarten, so dass keine eindeutigen Aussagen über die antiestrogenen Eigenschaften der applizierten Wirkstoffe gemacht werden können. Aus diesem Grund wurden geschlechtsreife, weibliche Ratten einer dreiwöchigen Wirkstoffbehandlung unterzogen. In diesem Versuch zeigten die beanspruchten Verbindungen eine ausgeprägte antiuterotrope Wirkung, die zum Teil über der Aktivität des Tamoxifens lag.

Zur Messung der mammatumorhemmenden Wirkung wurde von den beanspruchten Substanzen die Verbindung 1 (= «3-Hydroxy-Tamoxifen») ausgewählt und unter gleichen Testbedingungen mit Tamoxifen verglichen. Wie gezeigt werden konnte, erweist sich die beanspruchte Verbindung in der tumorhemmenden Aktivität dem Tamoxifen eindeutig überlegen.

Die erfindungsgemässen Verbindungen stellen somit eine wertvolle Bereicherung des Arzneimittelschatzes dar und können vor allem beim Menschen zur Behandlung des malignen Mammatumors eingesetzt werden.

Die Erfindung betrifft ausserdem Arzneimittel, die eine Verbindung der allgemeinen Formel (1) als Wirkstoff neben den üblichen pharmazeutischen Träger- und Hilfsstoffen enthalten.

Die beanspruchten Verbindungen werden bevorzugt oral verabreicht. Gewöhnlich beträgt die orale Tagesdosis 0.01 bis 0.2 g, vorzugsweise 0.02 bis 0.1 g. Trotzdem kann es gegebenenfalls erforderlich sein, von den genannten Mengen abzuweichen, und zwar in Abhängigkeit des individuellen Verhaltens gegenüber dem Medikament bzw. der Art seiner Formulierung und dem Zeitpunkt bzw. Intervall, zu welchem die Verabreichung erfolgt. So kann es in einigen Fällen ausreichend sein, mit weniger als der vorgenannten Mindestmenge auszukommen, während in anderen Fällen die genannte obere Grenze überschritten werden muss. Im Falle der Applikation grösserer Mengen kann es empfehlenswert sein, diese in mehreren Einzelgaben über den Tag zu verteilen.

Die Wirkstoffe können in üblicher Form zur oralen Verabreichung konfektioniert werden, z.B. in Kapseln, als Tabletten oder auch Dragees.

Durch Vermischen mit festen, pulverförmigen Trägerstoffen, wie Kartoffel- oder Maisstärke, mit Zusätzen wie Natriumcitrat, Calciumcarbonat und Bindemitteln wie Polyvinylpyrrolidon, Gelatine oder Cellulosederivaten, gegebenenfalls unter Zusatz von Gleitmitteln wie Magnesiumstearat, Natriumlaurylsulfat oder Polyethylenglykolen, können sie zu Tabletten oder zu Drageekernen verarbeitet werden. Selbstverständlich können bei den oralen Verabreichungsformen Geschmackskorrigenzien zugesetzt werden.

Als weitere Verabreichungsformen eignen sich Steckkapseln, z.B. aus Hartgelatine sowie geschlossene Weichgelatinekapseln mit einem Weichmacher z.B. Glycerin. Die Steckkapseln enthalten den Wirkstoff vorzugsweise als Granulat z.B. in Mischung mit Füllstoffen, wie Lactose, Saccharose, Mannit, Stärken wie z.B. Kartoffelstärke oder Amylopectin, Cellulosederivaten oder hochdispersen Kieselsäuren. In Weichgelatinekapseln ist der Wirkstoff vorzugsweise in geeigneten Flüssigkeiten, z.B. in Pflanzenölen oder flüssigen Polyethylenglykolen, gelöst oder suspendiert.

Im folgenden wird die Erfindung anhand von Ausführungsbeispielen näher beschrieben.

Beispiel 1

1-[4'-(2-Dimethylaminoethoxy)phenyl]-1-(3'-hydroxyphenyl)-2-phenyl-but-1-en

Herstellung der Vorstufen

a) 1-(4'-Methoxyphenyl)-2-phenyl-ethan-1-on

10.8 g (0.10 Mol) Methoxybenzol und 13.9 g 0.09 Mol) Phenylessigsäurechlorid in 1.0 l Methylenchlorid werden bei Raumtemperatur unter kräftigem Rühren portionsweise mit 13.3 g (0.10 Mol) Aluminiumchlorid versetzt und noch 2 Stdn. gerührt. Anschliessend wird das Reaktionsgemisch auf Eis gegossen und mit 50 ml Salzsäure versetzt. Nach Abtrennung der organischen Phase wird die wässrige Lösung zweimal mit je 500 ml Methylenchlorid ausgeschüttelt, die vereinigten organischen Phasen mit Wasser gewaschen und das Lösungsmittel i. Vak. entfernt. Der schmierige Rückstand wird mittels Wasserdampfdestillation von den flüchtigen Anteilen befreit und der verbleibende Feststoff nach Filtration und Waschen mit Wasser aus Ethanol kristallisiert. Farblose Kristalle vom Schmp. 75 °C; $R_f$ 0.7 [$CHCl_3$/$CH_3OH$ (9/1)]; Ausbeute: 18.3 g (90%).

($C_{15}H_{14}O_2$ (226.2)

$^1$H-NMR-Spektrum[1)]

($CDCl_3$):

3.77 s (3) $OC\underline{H}_3$

4.18 s (2) $C\underline{H}_2$

6.87 d (2) Aromaten-H [J = 9.0]
7.2 s (5) Aromaten-H
7.97 d (2) Aromaten-H [J = 9.0)

[1)]aufgenommen bei 60 MHz

die chemischen Verschiebungen sind in ppm gegen TMS (δ = 0.0) angegeben, die relativen Intensitäten sind in Klammern beigefügt.

s = Singulett; d = Dublett; t = Triplett; m = Multiplett.

J = Kopplungskonstante in Hz

b) 1-(4'-Methoxyphenyl)-2-phenyl-n-butan-1-on

2.4 g (0.10 Mol) Natriumhydrid werden in 300 ml stickstoffgesättigtem, wasserfreiem Dimethylformamid suspendiert und nach langsamer Zugabe von 22.6 g (0.10 Mol) 1-(4'-Methoxyphenyl)-2-phenyl-ethan-1-on in 50 ml wasserfreiem Dimethylformamid noch 1 Std. bei 40 °C gerührt. Anschliessend werden 13.1 g (0.12 Mol) Ethylbromid in 50 ml wasserfreiem Dimethylformamid bei 30 °C zugetropft und noch 2 Stdn. gerührt. Das Reaktionsgemisch wird mit 100 ml Wasser zersetzt, dann zweimal mit je 250 ml Ether ausgeschüttelt und die gesammelten Etherphasen sorgfältig mit Wasser gewaschen. Nach dem Trocknen über Natriumsulfat wird der Ether i. Vak. entfernt und der Rückstand aus Ethanol kristallisiert. Farblose Kristalle vom Schmp. 44 °C; $R_f$ 0.55 ($CH_2Cl_2$); Ausbeute: 24.7 g (97%).

$C_{17}H1_8O_2$ (254.3)
$^1$H–NMR-Spektrum
($CDCl_3$):

| | | |
|---|---|---|
| 0.87 | t | (3) C<u>H</u>$_3$ [J = 7.0] |
| 1.50 bis 2.53 | m | (2) C<u>H</u>$_2$ |
| 3.70 | s | (3) OC<u>H</u>$_3$ |
| 4.38 | t | (1) C<u>H</u> [J = 7.2] |
| 6.80 | d | (2) Aromaten-H [J = 9.0] |
| 7.23 | s | (5) Aromaten-H |
| 7.96 | d | (2) Aromaten-H [J = 9.0] |

c) 1-(4'-Hydroxyphenyl)-2-phenyl-n-butan-1-on

25.4 g (0.10 Mol) 1-(4'-Methoxyphenyl)-2-phenyl-n-butan-1-on und 34.5 g (0.30 Mol) Pyridinhydrochlorid werden geschmolzen und unter Rühren bei 220 °C 1 Std. unter Rückfluss erhitzt. Die noch flüssige Schmelze wird in Eiswasser gegossen und der Niederschlag mit 500 ml Ether aufgenommen. Die etherische Lösung wird nach dem Waschen mit Wasser, mit 1 N Natronlauge ausgeschüttelt. Die wässrig-alkalische Lösung wird mit 5 N Salzsäure angesäuert und mit 500 ml Ether extrahiert. Die organische Phase wird mit Wasser gewaschen und über Natriumsulfat getrocknet. Das Lösungsmittel wird i Vak. entfernt und das Rohprodukt aus verdünntem Ethanol kristallisiert. Farblose Kristalle vom Schmp. 131 °C; $R_f$ 0.45 [Toluol/Ethylacetat (9/1)]; Ausbeute: 16.3 g (68%).

$C_{16}H_{16}O_2$ (240.3)
$^1$H–NMR-Spektrum
($d_6$-Aceton):

| | | |
|---|---|---|
| 0,85 | t | (3) C<u>H</u>$_3$ [J = 7.0] |
| 1.37 bis 2.50 | m | (2) C<u>H</u>$_2$ |
| 2.73 bis 3.47 | breit | (1) O<u>H</u> [austauschbar mit $D_2O$] |
| 4.60 | t | (1) C<u>H</u> [J = 7.6) |
| 6.87 | d | (2) Aromaten-H [J = 9.0] |
| 7.33 | s | (5) Aromaten-H |
| 8.00 | d | (2) Aromaten-H [J = 9.0] |

d) 1-[4'-(2-Dimethylaminoethoxy)phenyl]-2-phenyl-n-butan-1-on

2.76 g (0.12 Mol) Natrium werden in 100 ml wasserfreiem Ethanol gelöst und 24 g (0.10 Mol) 1-(4'-Hydroxyphenyl)-2-phenyl-n-butan-1-on hinzugefügt. Die Lösung wird bei Rückflusstemperatur langsam mit 21.4 g (0.20 Mol) Dimethylaminoethylchlorid in 150 ml Toluol versetzt und das Reaktionsgemisch noch weitere 8 Stdn. unter Rückfluss erhitzt. Nach Abkühlung und Abtrennen der unlöslichen Anteile wird das Filtrat i. Vak. vom Lösungsmittel befreit und der Rückstand in 500 ml Ether aufgenommen. Die etherische Lösung wird mehrmals mit 2 N Natronlauge ausgeschüttelt und anschliessend mit Wasser gewaschen. Nach dem Trocknen über Natriumsulfat wird der Ether i. Vak. entfernt.

Farbloses Öl; $R_f$ 0.25 [$CHCl_3/CH_3OH$ (9/1)]; Ausbeute 19.3 g (62%).

$C_{20}H_{25}NO_2$ (311.4)
$^1$H–NMR-Spektrum
($CDCl_3$):

| | | |
|---|---|---|
| 0.88 | t | (3) C<u>H</u>$_3$ [J = 7.0) |
| 1.53 bis 2.83 | m | (2) C<u>H</u>$_2$ |
| 2.28 | s | (6) N(C<u>H</u>$_3$)$_2$ |
| 2.67 | t | (2) NC<u>H</u>$_2$ [J = 6.0] |
| 4.03 | t | (2) OC<u>H</u>$_2$ [J = 6.0] |
| 4.40 | t | (1) C<u>H</u> [J = 7.6] |
| 6.87 | d | (2) Aromaten-H [J = 8.4] |
| 7.23 | s | (5) Aromaten-H |
| 7.97 | d | (2) Aromaten-H [J = 8.4) |

e) 1-[4'-(2-Dimethylaminoethoxy)phenyl] -2-phenyl -1-[3'-(2-tetrahydropyranyloxy)phenyl]-n-butan-1-ol[Diastereomere]

42.2 g (0.15 Mol) 3'-(2-Tetrahydropyranyloxy)phenyl-magnesiumbromis in 200 ml wasserfreiem Tetrahydrofuran werden vorsichtig mit 31.1 g (0.10 Mol) 1-[4'-(2-Dimethylaminoethoxy)phenyl]-2-phenyl-n-butan-1-on in 100 ml wasserfreiem Tetrahydrofuran versetzt und anschliessend 2 Stdn. unter Rückfluss erhitzt. Die abgekühlte Reaktionslösung wird mit 150 ml gesättigter, wässriger Ammoniumchloridlösung zersetzt und mit 100 ml Ether ausgeschüttelt. Die organische Phase wird mit Wasser gewaschen und nach dem Trocknen über Natriumsulfat das Lösungsmittel i. Vak. entfernt. Der ölige Rückstand wird an Kieselgel mit Chloroform/Methanol (9/1) säulenchromatographisch von Verunreinigungen befreit und die reine Diastereomeren-Fraktion aus Petrolether kristallisiert. Farblose Kristalle vom Schmp. 56 °C; $R_f$ 0.50 [$CHCl_3/CH_3OH$ (9/1)]; Ausbeute 29.9 g (61%)

$C_{31}H_{39}NO_4$ (489.7)

| | | | |
|---|---|---|---|
| Ber. | C 76.04 | H 8.03 | N 2.86 |
| Gef. | C 76.28 | H 7.92 | N 2.79 |

Mol.-Gew. 489 (massenspektrometrisch bestimmt)

IR-Spektrum (KBr): ν (O–H) 3600 bis 3100 $cm^{-1}$

$^1H$–NMR-Spektrum ($d_6$-DMSO):

| | | |
|---|---|---|
| 0.6 breit | t | (3) $CH_3$ |
| 1.23 bis 1.97 | m | (8) $CH_2$ |
| 2.20 | s | (6) $N(CH_3)_2$ |
| 2.37 bis 2.77 | t | (2) $NCH_2$ |
| 3.27 bis 4.03 | m | (6) CH, 2 × $OCH_2$, OH |
| 5.45 breit | s | (1) OCH |
| 6.40 bis 7.43 | m | (13) Aromaten-H |

f) 1-[4'-(2-Dimethylaminoethoxy)phenyl]-1-(3'-hydroxyphenyl)-2-phenyl-n-butan-1-ol [Diastereomere]

49.0 g (0.1 Mol) der reinen Diastereomeren-Fraktion von 1-[4'-(2-Dimethylaminoethoxy)-phenyl]-2- phenyl-1- [3'- (2-tetrahydropyranyl-oxy)phenyl]-n-butan-1-ol in 500 ml Ethylacetat werden bei Raumtemperatur mit 200 ml 1-proz. wässriger Salzsäure versetzt und kräftig geschüttelt. Die Emulsion wird mit 5-proz. wässriger Ammoniaklösung neutralisiert und nach dem Absetzen, die wässrige Phase abgetrennt. Die organische Phase wird mit Wasser gewaschen und nach dem Trocknen über Natriumsulfat das Lösungsmittel i. Vak. ent fernt. Der Rückstand wird aus Ether/Petrolether (1/1) kristallisiert. Farblose Kristalle des Diastereomeren-Gemisches vom Schmp. 59 bis 60 °C; $R_f$ 0.35 [$CHCl_3/CH_3OH$ (7/3)]; Ausbeute 36.5 g (90%).

$C_{26}H_{31}NO_3$ (405.5)

| | | | |
|---|---|---|---|
| Ber. | C 77.01 | H 7.70 | N 3.45 |
| Gef. | C 76.81 | H 7.86 | N 3.38 |

Mol.-Gew. 405 (massenspektrometrisch bestimmt)

IR-Spektrum (KBr): ν (O–H) 3600 bis 2400 $cm^{-1}$

$^1H$–NMR-Spektrum ($d_6$-Aceton):

| | | |
|---|---|---|
| 0.7 | t | $CH_3$ [J = 7.8] |
| 0.87 | t | $CH_3$ [J = 7.6] |
| 1.43 | m | $CH_2$ |
| 2.23 | s | $N(CH_3)_2$ |
| 2.30 | s | $N(CH_3)_2$ |
| 2.37 bis 2.87 | m | $NCH_2$, |
| 3.47 bis 4.40 | m | $OCH_2$, CH |
| 6.20 bis 7.73 | m | Aromaten-H |

g) Erfindungsgemässe Herstellung von:

(E)-1-[4'- (2-Dimethylaminoethoxy)phenyl]-1-(3'-hydroxyphenyl) -2-phenyl-but-1-en-hydrochlorid

40.5 g (0.1 Mol) des Diastereomeren-Gemisches von 1-[4'-(2-Dimethylaminoethoxy)phenyl]-1- (3'-hydroxyphenyl)-2- phenyl-n-butan-1-ol in 500 ml Ethanol werden mit 25 ml konz. Salzsäure versetzt und 2 Stdn. unter Rückfluss erhitzt. Anschliessend wird das Lösungsmittel i. Vak. entfernt und der Rückstand aus Methanol/Ether (1/1) kristallisiert. Farblose Kristalle vom Schmp. 221 °C (Zers.); $R_f$ 0.25 [$CHCl_3/CH_3OH$ (7/3)]; Ausbeute: 20.3 g (48%).

$C_{26}H_{40}ClNO_2$ (423.9)

IR-Spektrum (KBr): ν (O–H,$^{\oplus}$NH) 3650 bis 2600 $cm^{-1}$

$^1H$–NMR-Spektrum ($d_4$-Methanol):

| | | |
|---|---|---|
| 0.9 | t | (3) $CH_3$ [J = 6.0] |
| 2.5 | q | (2) $CH_2$ [J = 6.0] |
| 2.97 | s | (6) $N(CH_3)_2$ |
| 3.5 | t | (2) $NCH_2$ [J = 5.0] |
| 4.27 | t | (2) $OCH_2$ [J = 5.0] |
| 6.53 bis 7.30 | m | (13) Aromaten-H |

h) Erfindungsgemässe Herstellung von

(E)-1-[4'-(2-Dimethylaminoethoxy)phenyl]-1-(3'-hydroxyphenyl)-2-phenyl-but-1-en

42.4 g (0.1 Mol) (E)-1-[4'-(2-Dimethylaminoethoxy)phenyl]-1-(3'-hydroxyphenyl)-2-phenyl-but-1-en-hydrochlorid werden in 200 ml verdünnter Ammoniaklösung suspendiert und zweimal mit je 250 ml Ethylacetat ausgeschüttelt. Die organische Phase wird mit Wasser neutral gewaschen und nach dem Trocknen über Natriumsulfat das Lösungsmittel i. Vak. entfernt. Der Rückstand wird aus Ether kristallisiert. Farblose Kristalle vom Schmp. 162 bis 163 °C; $R_f$ 0.40 [$CHCl_3/CH_3OH$ (7/3)]; Ausbeute: 37.2 g (96%).

$C_{26}H_{29}NO_2$ (387.5)

| | | | |
|---|---|---|---|
| Ber. | C 80.59 | H 7.54 | N3.61 |
| Gef. | C 80.50 | H 7.60 | N 3.55 |

Mol.-Gew. 387 (massenspektrometrisch bestimmt)

IR-Spektrum (KBr): ν (O–H) 3650 bis 3100 $cm^{-1}$

$^1H$–NMR'Spektrum ($d_6$-DMSO):

| | | |
|---|---|---|
| 0.83 | t | (3) $CH_3$ [J = 6.0] |
| 2.17 | s | (6) $N(CH_3)_2$ |
| 2.27 bis 2.73 | m | (4) $CH_2N$, $CH_2CH_3$ |
| 3.88 | t | (2) $OCH_2$ [J = 5.8] |
| 6.40 bis 7.37 | m | (13) Aromaten-H |
| 9.37 | s | (1) OH [austauschbar mit $D_2O$] |

i) (Z)-1-[4'-(2-Dimethylaminoethoxy)phenyl]-1-(3'-hydroxyphenyl]-2-phenyl-but-1-en

Der Rückstand der Mutterlauge des Kristallisates aus Beispiel 1 g wird in 200 ml verdünnter Ammoniaklösung suspendiert und zweimal mit je 250 ml Ethylacetat ausgeschüttelt. Die organische Phase wird mit Wasser gewaschen und nach dem Trocknen über Natriumsulfat das Lösungsmittel i. Vak. entfernt. Der Rückstand wird aus Methanol/Wasser (1/1) mehrmals kristallisiert. Farblose Kristalle vom Schmp. 173 °C; $R_f$ 0.40 [$CHCl_3/CH_3OH$ (7/3)]; Ausbeute: 7.7 g (20%).

$C_{26}H_{29}NO_2$ (387.5)

| | | | |
|---|---|---|---|
| Ber. | C 80.59 | H 7.54 | N 3.61 |
| Gef. | C 80.41 | H 7.53 | N 3.56 |

Mol.-Gew. 387 (massenspektrometrisch bestimmt)

IR-Spektrum (KBr): ν(O–H) 3650 bis 3100 $cm^{-1}$

$^1H$–NMR-Spektrum ($d_6$-DMSO):

| | | |
|---|---|---|
| 0.85 | t | (3) $CH_3$ [J=6.4] |
| 2.23 | s | (6) $N(CH_3)_2$ |
| 2.30 bis 2.80 | m | (4) $CH_2N$, $CH_2CH_3$ |
| 4.05 | t | (2) $OCH_2$ [J=5.8] |
| 6.10 bis 7.33 | m | (13) Aromaten-H |
| 9.03 | s | (1) OH [austauschbar mit $D_2O$] |

Beispiel 2a

Erfindungsgemässe Herstellung von (E)-1-[4'-(2-Diethylaminoethoxy)phenyl]-1-(3'-hydroxyphenyl)-2-phenyl-but-1-en

51.8 g (0.1 Mol) der analog Beispiel 1 e hergestellten reinen Diastereomeren-Fraktion von 1-[4'-(2-Diethylaminoethoxy)phenyl]-2-phenyl-1-[3'-(2-tetrahydropyranyloxy)phenyl]-n-butan-1-ol [hellgelbes Öl; $R_f$ 0.65 $CHCl_3/CH_3OH$ (9/1)] in 500 ml Ethanol werden mit 25 ml konz. Salzsäure versetzt, dann 2 Stdn. unter Rückfluss erhitzt und wie in den Beispielen 1 g bis 1 h beschrieben, aufgearbeitet. Farblose Kristalle vom Schmp. 121 °C [$CH_3OH/H_2O$ (1/1)]; $R_f$ 0.30 [$CHCl_3/CH_3OH$ (7/3)]; Ausbeute 10.4 g (25%).

$C_{28}H_{33}NO_2$ (415.6)

| | | | |
|---|---|---|---|
| Ber. | C 80.91 | H 8.00 | N 3.37 |
| Gef. | C 81.06 | H 8.07 | N 3.28 |

Mol.-Gew. 415 (massenspektrometrisch bestimmt)

IR-Spektrum (KBr): ν (O–H) 3600 bis 3100 $cm^{-1}$

$^1H$–NMR-Spektrum ($d_6$-DMSO):

| | | |
|---|---|---|
| 0.87 breit | t | (3) $CH_3$ |
| 0.97 | t | (6) $N(CH_2CH_3)_2$ [J=7.0] |
| 2.20 bis 2.87 | m | (8) $CH_2N(CH_2)_2$, $CH_2CH_3$ |
| 3.90 | t | (2) $OCH_2$ [J=7.0] |
| 6.27 bis 7.37 | m | (13) Aromaten-H |
| 9.20 breit | s | (1) OH [austauschbar mit $D_2O$] |

Beispiel 2 b

Z-1-[4'-(2-Diethylaminoethoxy)phenyl]-1-(3'-hydroxyphenyl)-2-phenyl-but-1-en

Farblose Kristalle vom Schmp. 156 °C [Methanol/Wasser (1/1)]; $R_f$ 0.30 [$CHCl_3/CH_3OH$ (7/3)]

$C_{28}H_{33}NO_2$ (415.6)

| | | | |
|---|---|---|---|
| Ber.: | C 80.91 | H 8.00 | N 3.37 |
| Gef.: | C 80.78 | H 8.00 | N 3.26 |

Mol.-Gew. 415 (massenspektrometrisch bestimmt)

IR-Spektrum (KBr): ν-OH 3600 bis 3100 $cm^{-1}$

$^1H$–NMR-Spektrum ($d_6$-DMSO):

| | | |
|---|---|---|
| 0.90 breit | t | (3) $CH_3$ |
| 1.01 | t | (6) $N(CH_2CH_3)_2$ [J=7.0] |
| 2.17 bis 3.00 | m | (8) $CH_2N(CH_2)$, $CH_2CH_3$ |
| 4.03 | t | (2) $OCH_2$ [J=7.0] |
| 6.17 bis 7.33 | | (13) Aromaten-H |
| 8.87 breit | s | (1) OH [austauschbar mit $D_2O$] |

Beispiel 3

(E)-1-(3'-Hydroxyphenyl)-1-[4'-(2-methylaminoethoxy)phenyl]-2-phenyl-but-1-en

Farblose Kristalle vom Schmp. 125 bis 127 °C (Ethanol); $R_f$ 0.15 [$CHCl_3CH_3OH$ (7/3)]

$C_{25}H_{27}NO_2$ (373.5)

| | | | |
|---|---|---|---|
| Ber.: | C 80.40 | H 7.29 | N 3.45 |
| Gef.: | C 80.55 | H 7.32 | N 3.61 |

Mol.-Gew. 373 (massenspektrometrisch bestimmt)

IR-Spektrum (KBr): ν (O–H; N–H) 3600 bis 2300 $cm^{-1}$;

$^1H$–NMR-Spektrum ($d_6$-DMSO):

| | | |
|---|---|---|
| 0.83 | t | (3) $CH_3$ [J=7.0] |
| 2.13 bis 2.70 | m | (2) $CH_2$ |
| 2.30 | s | (3) $NCH_3$ |
| 2.73 | t | (2) $NCH_2$ [J=5.6] |
| 3.83 | t | (2) $OCH_2$ [J=5.6] |
| 6.40 bis 7.43 | m | (13) Aromaten-H |

Beispiel 4

(E)-1-[4'-(2-Ethylaminoethoxy)phenyl]-1-(3'-hydroxyphenyl)-2-phenyl-but-1-en

Farblose Kristalle vom Schmp. 174 bis 175 °C (Aceton); $R_f$ 0.15 [$CHCl_3/CH_3OH$ (7/3)]

$C_{26}H_{29}NO_2$ (387.5)

| | | | |
|---|---|---|---|
| Ber.: | C 80.59 | H 7.54 | N 3.61 |
| Gef.: | C 80.55 | H 7.58 | N 3.67 |

Mol.-Gew. 387 (massenspektrometrisch bestimmt)

IR-Spektrum (KBr): ν (O–H) 3600 bis 3100 $m^{-1}$;
ν (N–H) 3300 $cm^{-1}$;

$^1H$–NMR-Spektrum ($d_6$-DMSO):

| | | |
|---|---|---|
| 0.85 | t | (3) $CH_3$ [J=7.0] |
| 0.98 | t | (3) $CH_3$ [J=7.2] |
| 2.13 bis 2.73 | m | (4) $NCH_2CH_3$, $CH_2CH_3$ |
| 2.80 | t | (2) $NCH_2$ [J=5.6] |
| 3.90 | t | (2) $OCH_2$ [J=5.6] |
| 6.43 bis 7.43 | m | (13) Aromaten-H |

Beispiel 5

(E)-1-[4'-(2-Dimethylaminoethoxy)phenyl]-1-(3'-hydroxyphenyl)-2-phenyl-but-1-en-hydrochlorid enthaltendes Arzneimittel

21.88 g pulverisiertes (E)-1-[4'-(2-Dimethylaminoethoxy)phenyl]-1-(3'-hydroxyphenyl)-2-phenyl-but-1-en-hydrochlorid werden mit 40 g Lactose und 140 g Stärke vermischt, anschliessend mit 33 g Talkum und 13 g Calciumstearat versetzt und nach sorgfältiger Durchmischung in zweitausend Hartgelatinekapseln geeigneter Grösse abgefüllt, so dass jede Kapsel 10 mg Wirkstoff (berechnet als freie Base) enthält.

Beispiel 6

(E)-1-[4'-(2-Ethylaminoethoxy)phenyl]-1-(3'-hydroxyphenyl)-2-phenyl-but-1-en enthaltendes Arzneimittel

20.0 g feinpulverisiertes (E)-1-[4'-(2-Ethylaminoethoxy)phenyl]-1-(3'-hydroxyphenyl)-2-phenyl-but-1-en werden nach dem Vermischen mit 111 g Mannit, 15 g Maisstärke und 6 g Alginsäure granuliert und das getrocknete Granulat nach sorgfältigem Vermischen mit 0.75 g Methylcellulose und 1.5 g Magnesiumstearat zu eintausend Tabletten verpresst, so dass jede Tablette 20 mg Wirkstoff enthält.

Pharmakologische Untersuchungen

a) Bindungsaffinität zum Estradiolrezeptor

Die Messung der Bindungsaffinität zum Estradiolrezeptor erfolgte nach der Methode von N. Devleeschouwer, G. Leclercq, A. Danguy und J.C. Heuson [Europ. J. Cancer, 14, 721–723 (1978)]. Das Uteruscytosol von weiblichen, präpubertären, weissen, 2 kg schweren Kaninchen (Neuseeländer) wurde 18 Std. bei 4 °C mit $2.5 \times 10^{-9}$ M [$^3$H]-Estradiol sowie jeweils unter Zusatz von unmarkiertem Estradiol (Kontrolle) bzw. Testsubstanz verschiedener Konzentration inkubiert. Die Bindungsaffinität zum Estradiolrezeptor wird ausgedrückt durch die, dem Uteruscytosol zugesetzte Konzentration an unmarkiertem Estradiol (Kontrolle) bzw. Testsubstanz, welche eine 50 proz. Verdrängung des am Estradiolrezeptor gebundenen [$^3$H]-Estradiol bewirkt.

Tabelle 3

Bindungsaffinität der Testsubstanzen

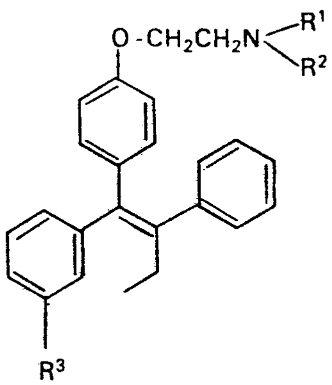

| Verbindung Nr. | $R^1$ | $R^2$ | $R^3$ | $ED_{50}$%$\times$ [M] |
|---|---|---|---|---|
| Estradiol (Kontrolle) | – | – | – | $1.3 \times 10^{-9}$ |
| Tamoxifen | $CH_3$ | $CH_3$ | H | $3.8 \times 10^{-7}$ |
| 1 | $CH_3$ | $CH_3$ | OH | $2.4 \times 10^{-8}$ |
| 2 | $C_2H_5$ | $C_2H_5$ | OH | $6.5 \times 10^{-8}$ |
| 3 | $CH_3$ | H | OH | $3.7 \times 10^{-8}$ |

x) Konzentration der Substanz, die 50% [$^3$H]-Estradiol vom Estradiol-Rezeptor verdrängt.

b) Antiuterotrope Wirkung

Die antiuterotrope Wirkung wurde nach einem modifizierten «Dorfman Test» [R.I. Dorfman, Methods in Hormone Research II, S. 707, Academic Press, New York – London, 1962] an geschlechtsreifen, weiblichen Sprague-Dawley-Ratten bestimmt.

Die Testverbindungen wurden in 0.25proz. wässriger Agarsuspension aufgenommen und per Schlundsonde über einen Zeitraum von 21 Tagen sechsmal wöchentlich verabreicht. Am Versuchsende wurde das Uterusgewicht der mit Wirkstoff behandelten Tiere in Beziehung gesetzt zum Uterusgewicht von Kontrolltieren, die nur eine leere Agarsuspension erhielten.

Tabelle 4

Antiuterotrope Aktivität der Testsubstanzen

| Verbindung Nr. | Zahl der Versuchstiere | Dosis mg/kg/Tag | Uterusgewicht gegenüber Kontrolltieren |
|---|---|---|---|
| Tamoxifen | 10 | 3 | −40% |
| 1 | 10 | 3 | −42% |
| 2 | 10 | 3 | −39% |
| 3 | 10 | 3 | −53% |

c) Mammatumorhemmende Wirkung

Die tumorhemmende Wirkung wurde am Modell des mit 7,12-Dimethylbenz[a]anthracen induzierten Mammatumors der weiblichen Sprague-Dawley-Ratte (Stamm Hannover) nach der Methode von M.J. Golder [Europ. J. Cancer 11, 571 (1975)] und D.P. Griswold et al [Cancer Research 26, 2169 (1966)] bestimmt.

Die Testverbindungen wurden in 0.25proz. Agarsuspension aufgenommen und per Schlundsonde über einen Zeitraum von 28 Tagen sechsmal wöchentlich verabreicht. Zweimal wöchentlich und am 28. Versuchstag wurde die Zahl der Tiere festgestellt und die Tumorfläche ($mm^2$/Tier) der Therapie- und Kontrolltiere gemessen. Bestimmt wurde am Versuchsende die prozentuale Zunahme der durchschnittlichen Tumorfläche der behandelten Tiere im Vergleich zu den Kontrolltieren, die als 100% angenommen wurde.

Tabelle 5
Tumorhemmende Aktivität der Testsubstanzen

| Verbindung Nr. | Zahl der Versuchstiere | Dosis mg/kg/Tag | Relative Zunahme der durchschnittlichen Tumorfläche |
|---|---|---|---|
| Leerkontrolle | 10 | – | 100% |
| Tamoxifen | 12 | 3 | 35% |
| 1 | 12 | 3 | 23% |

**Patentansprüche**

1. 1,1,2-Triphenyl-but-1-en-Derivate der allgemeinen Formel (1)

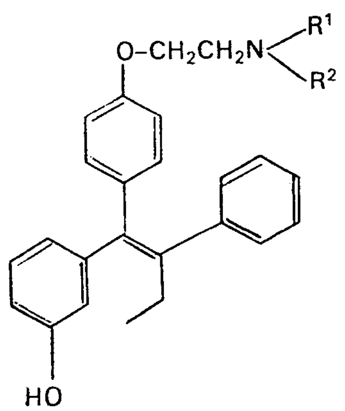

(1)

in der $R^1$ und $R^2$ gleich oder verschieden sein können, wobei im Falle $R^1$ gleich $R^2$ die Substituenten jeweils Methyl- oder Ethylgruppen bedeuten, während im Falle $R^1$ ungleich $R^2$, ein Substituent Wasserstoff bedeutet und der andere Substituent eine Methyl- oder Ethylgruppe darstellt und deren therapeutisch verträgliche Salze.

2. Verfahren zur Herstellung von 1,1,2-Triphenyl-but-1-en-Derivaten nach Anspruch 1, dadurch gekennzeichnet, dass man Carbinole der allgemeinen Formel (2)

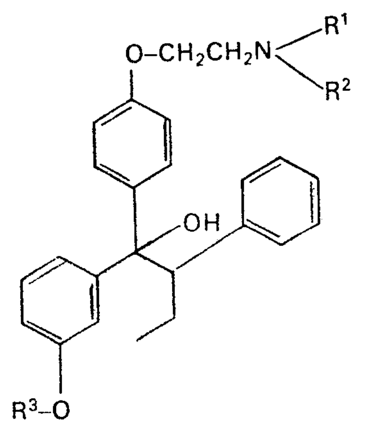

(2)

in der $R^1$ und $R^2$ gleich oder verschieden sein können, wobei im Falle $R^1$ gleich $R^2$ die Substituenten jeweils Methyl- oder Ethylgruppen bedeuten, während im Falle $R^1$ ungleich $R^2$ ein Substituent eine Benzylgruppe darstellt und der andere Substituent eine Methyl oder Ethylgruppe sein kann und $R^3$ Wasserstoff oder eine leicht hydrolysierbare Schutzgruppe ist, in an sich bekannter Weise durch Einwirken von Mineralsäure, gegebenenfalls unter Abspaltung der Schutzgruppe dehydratisiert, aus dem anfallenden Isomerenpaar die E-Form durch Kristallisation isoliert und eine gegebenenfalls vorhandene Benzylgruppe hydrogenolytisch abspaltet.

3. Arzneimittel enthaltend eine Verbindung gemäss Anspruch 1 als Wirkstoff, sowie übliche Träger- und Hilfsstoffe.

**Revendications**

1. Dérivés de 1,1,2-triphényl-but-1-ène de formule générale (1)

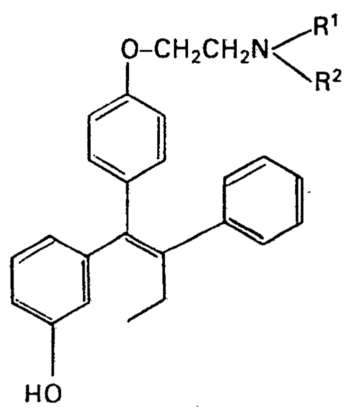

(1)

où $R^1$ et $R^2$ peuvent être semblables ou différents, où dans le cas où $R^1$ est semblable à $R^2$ les substituants représentent à chaque fois des groupes méthyle ou éthyle, tandis que dans le cas où $R^1$ est différent de $R^2$, un substituant représente un hydrogène et l'autre substituant représente un groupe méthyle ou éthyle, et leurs sels thérapeutiquement acceptables.

2. Procédé de préparation de dérivés de 1,1,2-triphényl-but-1-ène selon la revendication 1, caractérisé en ce qu'on déshydrate des carbinols de formule générale (2)

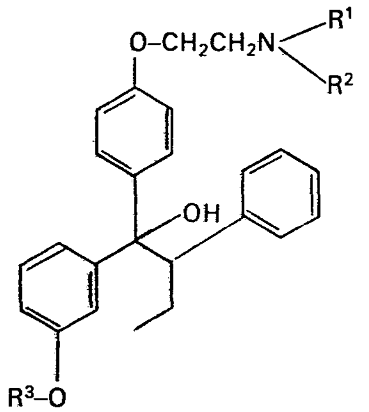

(2)

où $R^1$ et $R^2$ peuvent être semblables ou différents, où dans le cas où $R^1$ est semblable à $R^2$ les substituants représentent à chaque fois des groupes méthyle ou éthyle, tandis qu'au cas où $R^1$ est différent de $R^2$ un substituant représente un groupe benzyle et l'autre substituant peut être un groupe méthyle ou éthyle et $R^3$ est un hydrogène ou un groupe protecteur facilement hydrolysable, de façon connue par action d'un acide minéral, éventuellement avec séparation du groupe protecteur, en ce qu'on isole de la paire d'isomères apparue la forme E par cristallisation et en ce qu'on sépare par hydrogénolyse un groupe benzyle éventuellement présent.

3. Médicament contenant un composé selon la revendication 1 comme substance active, ainsi que des supports et additifs habituels.

**Claims**

1. 1,1,2-triphenyl-but-1-ene derivatives of the general formula (1)

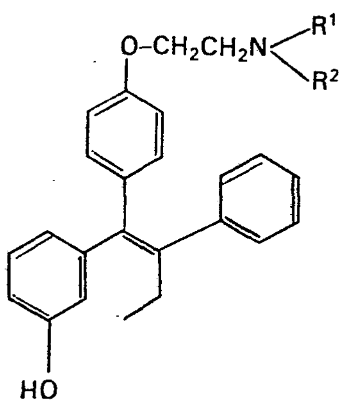

(1)

in which $R^1$ and $R^2$ can be the same or different, in the case of $R^1 = R^2$, the substituents each being methyl or ethyl groups, in the case of $R^1$ being different to $R^2$, one substituent being hydrogen and the other substituent being a methyl or ethyl group and their therapeutically acceptable salts.

2. A process for the preparation of 1,1,2-triphenyl-but-1-ene derivatives according to claim 1, characterised in that carbinols of the general formula (2)

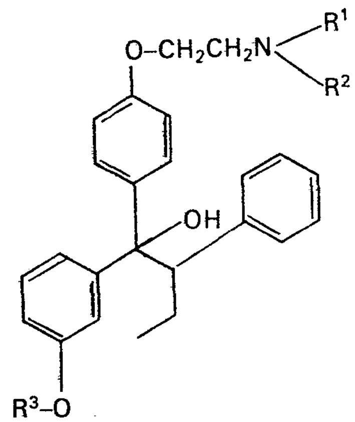

(2)

in which $R^1$ and $R^2$ can be the same or different, in the case of $R^1 = R^2$, the substituents each being methyl or ethyl groups while in the case of $R^1$ being different to $R^2$, one substituent is a benzyl group and the other substituent can be a methyl or ethyl group and $R^3$ is hydrogen or an easily hydrolisable protective group, are dehydrated in a manner known per se by reacting a mineral acid, optionally with removal of the protective group, the E-form is isolated from the resulting isomer pair by crystallisation and a benzyl group optionally present is removed hydrogenolytically.

3. A drug containing a compound according to claim 1 as active ingredient and usual carrier substances and auxiliary agents.